# EUROPÄISCHE PATENTANMELDUNG

(11) **EP 2 630 934 A1**
(43) Veröffentlichungstag der Anmeldung: **28.08.2013**
(21) Anmeldenummer: 13000816.2
(22) Anmeldetag: 18.02.2013
(51) Int. Cl.: A61F 2/12, G01M 3/18, A61B 5/053

(54) **Brustimplantat mit elektronischer Überwachung**

(30) Priorität: 22.02.2012 DE 202012001796 U
(71) Anmelder: Osypka, Peter, 79639 Grenzach-Wyhlen (DE)
(72) Erfinder: Osypka, Peter, 79639 Grenzach-Wyhlen (DE)

(57) **Zusammenfassung**

Die Erfindung betrifft somit ein Brustimplantat (1) bestehend aus einem Implantat-Körper (12) und einer Schutzhülle (4) wobei der Implantat-Körper (12) aus einer flexiblen aber undurchlässigen Hülle (2) mit einer darin befindlichen elektrisch leitenden Füllung (10) besteht, dadurch gekennzeichnet, dass der Implantat-Körper (12) separat in einer flexiblen aber undurchlässigen Schutzhülle (4) eingebettet ist und von der Schutzhülle (4) völlig umgeben wird und dass zwischen der Hülle (2) und der Schutzhülle (4) sich eine elektrisch leitende physiologische Flüssigkeit befindet und dass die Schutzhülle (4) eine Außenseite (4a) und eine Innenseite (4b) hat und dass die Außenseite (4a) mindestens einen elektrischen Pol oder eine Elektrode (6) aufweist und die Innenseite (4b) mindestens einen elektrischen Pol oder eine Elektrode (7) aufweist, wobei die beiden Pole oder Elektroden (6) und (7) mit einer elektrischen Widerstands- oder Impedanz-Messvorrichtung (5) verbunden sind und wobei die Innenseite (4b) oder die Hülle (2) auf ihrer der Schutzhülle (4) zugewandten Seite mindestens zwei elektrische Pole oder Elektroden (8) und (11) aufweist, die in einer gesonderten Schaltung mit einer elektrischen Widerstands- oder Impedanz-Messvorrichtung (5) verbunden sind.

## Beschreibung

Die Erfindung betrifft ein medizinisches in den Körper des Menschen einsetzbares Implantat, insbesondere ein Brustimplantat, wobei die elektronische Überwachung der Unversehrtheit des Implantats ermöglicht wird.

Brustimplantate bestehen im Wesentlichen aus einer äußeren flexiblen, undurchlässigen Hülle aus dem Werkstoff Silikon und einer darin befindlichen gelartigen Füllung ebenfalls aus Silikon aber mit einer anderen Konsistenz.

Derartige Implantate sind seit langem bekannt. Beispielshaft wird auf die Patentveröffentlichungen US4,795, 463; US5,496, 367; US6,755,861 verwiesen.

Trotz sorgfältiger Herstellung und der Verwendung von biokompatiblem Material kann es beispielsweise bei Unfällen dazu kommen, dass die Hülle des Implantates stellenweise durchlässig wird, einen Riss bekommt und die innere Füllung austreten kann. Das auslaufende Material kann zu erheblichen medizinischen Komplikationen führen. Neben ästhetischen Beeinträchtigungen wie Deformationen bzw. Verhärtungen des Füllmaterials, sind oft chirurgische Eingriffe notwendig, um größeren Schaden von der Trägerin abzuwenden. Es ist daher von großer Wichtigkeit, dass ein Schaden am Implantat so früh wie möglich angezeigt wird. Das ist aber nur möglich wenn die Trägerin selbst z.B. täglich auf einfache Weise überprüfen kann, ob das Implantat noch in Ordnung ist oder ein bereits beginnender Schaden messbar ist.

Die US-Patentveröffentlichung US2009/0012372 (WO2008/055229) beschreibt einen Sensor mit dessen Hilfe ein Riss in der Außenhülle eines Brustimplantats durch ein elektronisches Signal nachgewiesen werden kann. Das Signal zeigt an, dass bereits Silikon-Gel in den Körper gelangt ist. Werden derartige Ereignisse zu spät erkannt so sind langwierige, kostenintensive und manchmal auch tödliche Folgen nicht auszuschließen.

Die europäische Patentanmeldung EP2329795 beschreibt ein Brustimplantat mit einer flexiblen, aber undurchlässigen Hülle und mit einer darin befindlichen elektrisch leitfähigen Silikonfüllung. Schäden an der Hülle, die dazu führen dass die Silikonfüllung austritt werden durch eine Widerstandsmessung festgestellt. Tritt bei einer Perforation der Hülle des Implantats elektrisch leitfähige Füllung nach außen schließt sich der elektrische Kontakt zwischen einer im Inneren des Implantats angebrachten Innenelektrode und einer an der Außenseite angebrachten Außenelektrode. Dabei verzeichnet eine Widerstandsmessvorrichtung mit der die Innen- und die Außenelektrode verbunden sind, einen Abfall des Widerstands.

Der Nachteil dieser Messmethoden ist, dass der Schaden erst erkannt wird, wenn bereits ein Riss im Implantat vorliegt und Füllmaterial in den Körper gelangt ist.

Es besteht daher die Aufgabe ein Implantat zu schaffen, bei welchem die Unversehrtheit des Implantats überwacht werden kann und dadurch das Austreten von Implantats-Füllung in den Körper vermieden wird.

Die Lösung ist ein Implantat, das über eine Doppelhülle verfügt, wobei die innere Hülle, die das eigentliche Implantat enthält separat in einer elastischen äußeren Hülle eingebettet ist, in der sich eine physiologische Flüssigkeit wie z.B. Kochsalzlösung befindet. Es findet eine doppelte elektronische Überwachung statt. Erfolgt ein Riss in der Außenhülle, so fließt physiologische Kochsalzlösung in den Körper und zeigt gleichzeitig einen Schaden am Gesamtimplantat an. Auch bei einem Riss an der Innenhülle wird ein Fehler signalisiert. Schließich wird auch ein Riss in beiden Hüllen mit einem Ausfluss in den Körper des Trägers als Schaden signalisiert.

Die Erfindung betrifft somit ein Brustimplantat (1) bestehend aus einem Implantat-Körper (12) und einer Schutzhülle (4) wobei der Implantat-Körper (12) aus einer flexiblen aber undurchlässigen Hülle (2) mit einer darin befindlichen elektrisch leitenden Füllung (10) besteht, **dadurch gekennzeichnet, dass** der Implantat-Körper (12) separat in einer flexiblen aber undurchlässigen Schutzhülle (4) eingebettet ist und von der Schutzhülle (4) völlig umgeben wird und dass zwischen der Hülle (2) und der Schutzhülle (4) sich eine elektrisch leitende physiologische Flüssigkeit befindet und dass die Schutzhülle (4) eine Außenseite (4a) und eine Innenseite (4b) hat und dass die Außenseite (4a) mindestens einen elektrischen Pol oder eine Elektrode (6) aufweist und die Innenseite (4b) mindestens einen elektrischen Pol oder eine Elektrode (7) aufweist, wobei die beiden Pole oder Elektroden (6) und (7) mit einer elektrischen Widerstands- oder Impedanz-Messvorrichtung (5) verbunden sind und wobei die Innenseite (4b) oder die Hülle (2) auf ihrer der Schutzhülle (4) zugewandten Seite mindestens zwei elektrische Pole oder Elektroden (8) und (11) aufweist, die in einer gesonderten Schaltung mit einer elektrischen Widerstands- oder Impedanz-Messvorrichtung (5) verbunden sind.

Da die beiden Pole oder Elektroden (6) und (7) durch die Schutzhülle (4) elektrisch voneinander getrennt sind, besteht eine sehr hohe Impedanz oder ein sehr hoher Widerstand.

Ebenso sind die beiden Pole (8) und (11) gegeneinander isoliert, sodass im Normalfall der durch die physiologische Lösung vorgegebene Widerstand gemessen wird

Die beiden Pole oder Elektroden (8) und (11) sind vorzugsweise in einer aus Silikon geformten Rille (9) eingebettet und so durch die Silikonrille gegeneinander isoliert.

Die Rille (9) mit den Elektroden (8) und (11) befindet sich entweder an der Innenseite (4b) der Schutzhülle (4) oder an der Seite der Schutzhülle (2), die mit der Kochsalzlösung in Kontakt steht, also der der Schutzhülle (4) zugewandten Seite.

Die physiologische Lösung ist vorzugsweise physiologische Kochsalzlösung (NaCl 0.9%).

Die elektrisch leitende Füllung (10) ist vorzugsweise eine Silikonfüllung mit einem leitfähigen Zusatz wie beispielsweise Carbon- oder Metallpulver.

Die flexible aber undurchlässigen Hülle (2) und die flexible aber undurchlässigen Hülle (4) bestehen vorzugsweise aus demselben elektrisch nicht leitenden Material, beispielsweise aus besonders dehnbarem Silikon.

### Defekt 1

Tritt bei einer Perforation der Hülle (2) aber intakter Schutzhülle (4) leitfähiges Silikon aus, so mischt sich das Silikon mit der Kochsalzlösung und führt zu einer Änderung des elektrischen Widerstands zwischen den Polen (8) und (11). Zwischen den Elektroden (6) und (7) tritt durch die intakte Isolierung durch die Schutzhülle (4) keine Widerstandsänderung ein.

### Defekt 2

Tritt bei einer Perforation der Schutzhülle (4) aber intakter innerer Hülle (2) des Implantats Kochsalzlösung aus, so ändert sich der elektrische Widerstand zwischen den Polen (6) und (7).

Die Widerstandsänderung dient als Signal für die Fehlerhaftigkeit oder Beschädigung des Implantats. Die Anzeige des Widerstands- bzw. Impedanz-Abfalls könnte beispielsweise mittels eines akustischen Signals oder eines Vibrationsalarms oder eines Lichtsignals erfolgen.

Das Austreten der physiologischen Kochsalzlösung ruft keine Schäden hervor, zeigt aber ein Schaden am Gesamtimplantat an, noch bevor die Hülle (2) beschädigt ist. Es wird also eine Vorwarnung abgegeben, die es dem Implantat-Träger ermöglich zu reagieren bevor Schaden eintritt.

Für eine möglichste einfache Kontrolle des Implantats und der dafür notwendigen Übermittlung der Änderung der Widerstands-oder Impedanz-Werte ist es besonders günstig, wenn die beiden Pole oder Elektroden (6) und (7) sowie die Pole oder Elektroden (8) und (11) mit einem die Messvorrichtung enthaltenen Transponder (5) verbunden sind, der zur

Übertragung der Messwerte auf einen externen Empfänger ausgebildet ist. Die Elektroden (6) und (7) und (8) und (11) stellen gesonderte Schaltkreise dar.

Der Transponder ist vorzugsweise ein RFID Transponder und enthält mindestens eine Widerstandsmessbrücke.

Die Pole oder Elektroden (6) und (7) befinden sich vorzugsweise an gegenüberliegenden Außenseiten des Transponders (5) und bilden mit diesem eine Einheit.

Somit genügt es für die Überprüfung des Implantats beispielsweise bei dem Verdacht, dass eine Beschädigung seiner Hülle vorliegt, einen externen Empfänger in die Reichweite des Transponders zu bringen und die Widerstands- oder Impedanz-Werte abzulesen. Somit kann die Trägerin des Implantats jederzeit mit einem kleinen Gerät (ähnlich wie ein Handy) dass sie vor ihre Brust hält, per Knopfdruck über einen sogenannten RFID Transponder feststellen, ob ihr Implantat in Ordnung ist. Die Messung dauert höchstens paar Sekunden.

Um die an die natürliche Anatomie angepasste Form des eingesetzten Implantats trotz der angebrachten Pole oder Elektroden, des Transponders und der Messvorrichtung zu bewahren, ist es besonders günstig, wenn die elektrischen Pole oder die Elektroden (6,7,8,11) an der in Gebrauchsstellung rückwärtigen Wandung oder Rückseite (20) des Implantats angeordnet oder befestigt ist. Unter Rückseite wird dabei die in Gebrauchslage von der Körperaußenseite abgewandte Seite, bei einem Brustimplantat die den Rippen zugewandte Seite, verstanden.

Besonders günstig ist es, wenn die die außenliegende Elektrode (6) aufnehmenden Stelle oder Wandung eine Vertiefung für den Pol oder die Elektrode aufweist und der Pol oder die Elektrode in Gebrauchsstellung gegenüber der Oberfläche der Außenseite wenigstens teilweise oder derart eingesenkt ist, dass die Außenseite des Pols oder der Elektrode mit der Außenseite der ihn aufnehmenden Wandung bündig ist.

Damit ragt die Außenelektrode (6) nur wenig oder gar nicht aus der Oberfläche des Implantats heraus und unerwünschte Veränderungen hinsichtlich seiner bestimmungsgemäßen Form durch die Außenelektrode können vermieden werden.

Eine für die Anwendung des Implantats vorteilhafte Ausgestaltung der Erfindung kann darin bestehen, dass an der Rückwand (20) des Implantats eine Öffnung für das Einbringen der Implantats-Füllung und ein Verschluss dafür vorgesehen ist und dass der außenliegende Pol oder die Elektrode, der Transponder und der innenliegende Pol oder die Elektrode an oder in dem Verschluss angeordnet sind.

Nachstehend sind Ausführungsbeispiele der Erfindung anhand der Zeichnungen näher beschrieben. Es zeigt in zum Teil schematisierter Darstellung:
Fig. 1 eine Seitenansicht eines erfindungsgemäßen Implantats in Gebrauchsstellung und eine Sende-Empfänger-Einheit zum Zusammenwirken mit einem Transponder.
Fig. 2 eine detaillierte Seitenansicht gemäß Fig. 1
Fig. 3 eine Vorderansicht eines erfindungsgemäßen Implantats

### Beschreibung der Abbildungen

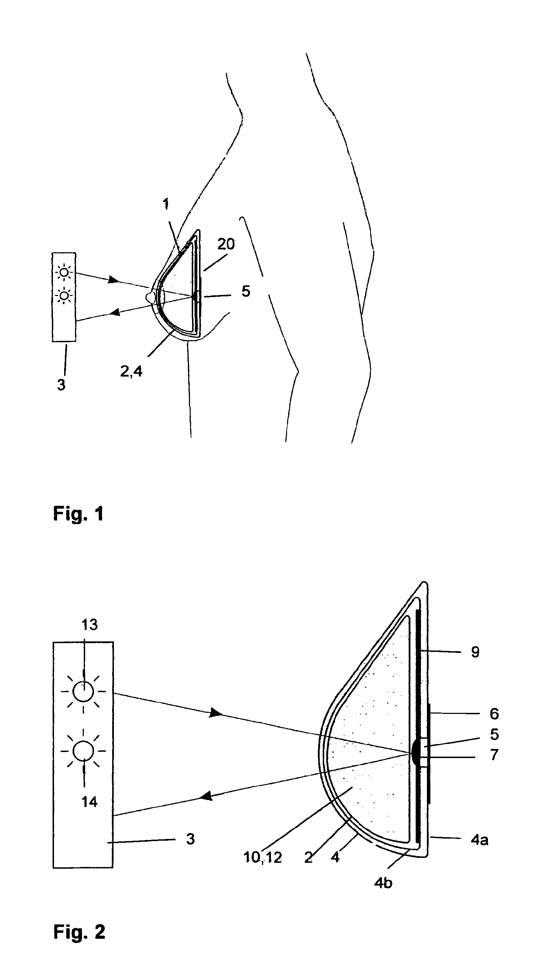

Fig. 1 zeigt eine Seitenansicht eines erfindungsgemäßen Implantats (1) mit einer in Gebrauchsstellung rückwärtigen Wand (20) und den undurchlässigen Hüllen (2,4) und eine Sender-Empfänger-Einheit (3) zum Zusammenwirken mit einem Impedanz-Messgerät mit einer integrierten RFID (radio frequency indication) Schaltung (5) (im folgenden Transponder (5) genannt.

Der Transponder (5) ist hier ein passiver Transponder, verfügt also über keine eigene Stromversorgung, sondern wird über das elektromagnetische Feld der Sender-Empfängereinheit gespeist. Sobald er angeregt wird kann er die gemessene Impedanz- oder Widerstandswerte an den externen Sender-Empfänger (3) übertragen. Die Sender-Empfänger-Einheit besitzt vorzugsweise eine grüne und eine rote Leuchtdiode LED die Änderungen in den Widerstandwerten anzeigen.

Es ist aber auch möglich, dass der Transponder über eine eigene, hier nicht weiter dargestellte Energieversorgung, zum Beispiel eine Batterie verfügt. Der Transponder kann folglich auch ohne Anregung beziehungsweise Speisung aus dem elektromagnetischen Feld einer Sender-Empfängereinheit (3) Daten und Messwerte an einen externen Empfänger übertragen.

Fig. 2 zeigt eine vergrößerte Seitenansicht gemäß Fig. 1 mit den beiden Hüllen (2) und (4). In der undurchlässigen inneren Hülle (2) befindet sich eine elektrisch leitfähige Füllung (10), vorzugsweise eine Silikonfüllung mit einem leitfähigen Zusatz wie beispielsweise Carbon-oder Metallpulver. Diese Hülle (2) mit Füllung (10) stellt den Implantat-Körper (12) dar.

Die ebenfalls undurchlässige äußere Hülle (4), die die Doppelhülle oder Schutzhülle darstellt, ist mit leitfähiger Kochsalzlösung gefüllt. Zwischen der Hülle (2) und der Hülle (4) befindet sich die Kochsalzlösung.

Bei der Schutzhülle (4) unterscheidet man eine Außenseite (4a) und eine Innenseite (4b). Die Außenelektrode (6) befindet sich auf der Schutzhülle (4) auf der Außenseite (4a). Die Elektrode (6) kann beispielsweise auf die Außenseite (4a) geklebt werden. Die Innenelektrode (7) befindet sich an der Innenseite (4b). Die Elektrode (7) kann beispielsweise auf die Innenseite (4b) geklebt werden. Sie liegt vorzugsweise der Außenelektrode (6) gegenüber.

Die Elektroden (6) und (7) sind durch die äußere Hülle (4) voneinander elektrisch getrennt und/oder zusätzlich gegeneinander isoliert. Die beiden Elektroden (6) und (7) sind mit einer nicht gesondert dargestellten elektrischen Widerstands- oder einer Impedanz-Messvorrichtung verbunden. Die Messvorrichtung kann in einen Transponder (5) integriert sein.

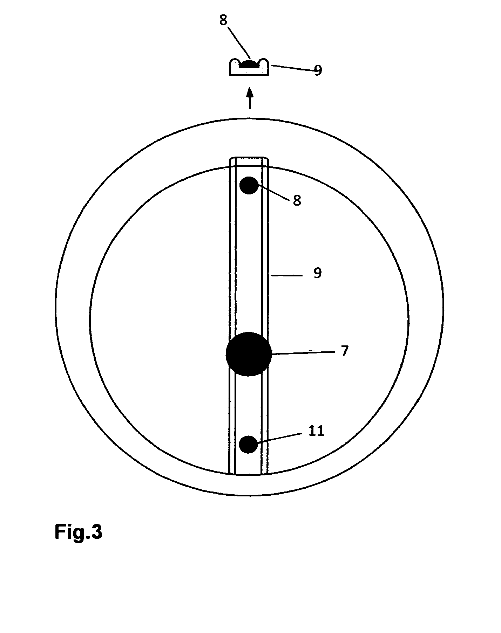

An der Innenseite (4b) der Schutzhülle (4) ist eine aus Silikon geformte Rille (9) befestigt, vorzugsweise angeklebt. Die Rille 9 trägt die Elektroden (8) und (11), siehe Fig. 3, und gegebenenfalls die Elektrode (7).

In der in Fig. 2 dargestellten Ausführungsform befinden sich die Elektroden (6) und (7) an gegenüberliegenden Außenseiten des Transponders (5) und bilden mit diesem eine Einheit. Die Elektrode (7) kann auch in die Rille (9) eingebettet sein oder sich auf der Rille (9) befinden.

Diese Einheit (Transponder/Elektroden) wird so an beziehungsweise in der Schutzhülle (4) befestigt, dass die Innenelektrode (7) der Hülle (2) zugewandt ist und die Außenelektrode (6) an der der Hülle (2) abgewandten Seite des Transponders (5) sitzt.

Durch die Hülle (4) und den Transponder (5) werden die Elektroden (6) und (7) elektrisch voneinander getrennt.

Tritt bei einer Perforation der Schutzhülle (4) aber intakter innerer Hülle (2) des Implantats Kochsalzlösung aus, so vermindert sich der elektrische Widerstand zwischen den Polen (6) und (7). Der Transponder (5) überträgt die gemessene Impedanz- oder Widerstandswerte an den externen Sender-Empfänger (3).. In der in Fig. 2 dargestellten Ausführungsform zeigt der Sender -Empfänger (3) Widerstandsänderung durch Aufleuchten der roten LED Leuchte (13) an.

Fig. 3 zeigt eine Vorderansicht des erfindungsgemäßen Implantats mit den Elektroden (8) und (11), die in einer aus Silikon geformten Rille (9) eingebettet sind, damit die Elektroden von der Kochsalzlösung gut umspült sind. Die Rille ist eine U-förmige Vertiefung im Silikon.

Die beiden Elektroden (8) und (11) sind mit einer nicht gesondert dargestellten elektrischen Widerstands- oder einer Impedanz-Messvorrichtung verbunden. Vorzugsweise ist diese Messvorrichtung auch in den Transponder (5) integriert.

Tritt bei einer Perforation der Hülle (2) aber intakter Schutzhülle (4) leitfähiges Silikon zwischen Hülle (2) und Schutzhülle (4) so ändert sich der elektrische Widerstand zwischen den Elektroden (8) und (11) da sich das Silikon mit der Kochsalzlösung vermischt.

Die Widerstandsänderung zwischen den Elektroden (8) und (11) wird durch den Sender (3) bei seiner Aktivierung als Alarm mit der roten LED-leuchte angezeigt.

Ist das Implantat unversehrt so leuchtet beim Aktivieren des Senders (3) die grüne LED-Leuchte (14) auf.

## Patentansprüche

1. Brustimplantat (1) bestehend aus einem Implantat-Körper (12) und einer Schutzhülle (4) wobei der Implantat-Körper (12) aus einer flexiblen aber undurchlässigen Hülle (2) mit einer darin befindlichen elektrisch leitenden Füllung (10) besteht, **dadurch gekennzeichnet, dass** der Implantat-Körper (12) separat in einer flexiblen aber undurchlässigen Schutzhülle (4) eingebettet ist und von der Schutzhülle (4) völlig umgeben wird und dass zwischen der Hülle (2) und der Schutzhülle (4) sich eine elektrisch leitende physiologische Flüssigkeit befindet und dass die Schutzhülle (4) eine Außenseite (4a) und eine Innenseite (4b) hat und dass die Außenseite (4a) mindestens einen elektrischen Pol oder eine Elektrode (6) aufweist und die Innenseite (4b) mindestens einen elektrischen Pol oder eine Elektrode (7) aufweist, wobei die beiden Pole oder Elektroden (6) und (7) mit einer elektrischen Widerstands- oder Impedanz-Messvorrichtung (5) verbunden sind und wobei die Innenseite (4b) oder die Hülle (2) auf ihrer der Schutzhülle (4) zugewandten Seite mindestens zwei elektrische Pole oder Elektroden (8) und (11) aufweist, die in einer gesonderten Schaltung mit einer elektrischen Widerstands- oder Impedanz-Messvorrichtung (5) verbunden sind.

2. Implantat (1) nach Anspruch 1, **dadurch gekennzeichnet dass** die beiden Pole der Elektroden (6) und (7) mit einem die Messvorrichtung enthaltenden Transponder (5) verbunden sind, der zur Übertragung der Messwerte auf einen externen Empfänger (3) ausgebildet ist.

3. Implantat (1) nach Anspruch 1, **dadurch gekennzeichnet dass** die beiden Pole der Elektroden (8) und (11) mit einem die Messvorrichtung enthaltenden Transponder (5) verbunden sind, der zur Übertragung der Messwerte auf einen externen Empfänger (3) ausgebildet ist.

4. Implantat (1) nach Anspruch 2 oder 3, **dadurch gekennzeichnet, dass** der Transponder (5) mindestens eine Widerstandsmessbrücke enthält.

5. Implantat (1) nach Anspruch 2 -4, **dadurch gekennzeichnet, dass** der Transponder (5) ein RFID Transponder ist.

6. Implantat (1) nach Anspruch 1, **dadurch gekennzeichnet, dass** die beiden Pole oder Elektroden (6) und (7) durch die Schutzhülle (4) voneinander elektrisch getrennt und/oder zusätzlich gegeneinander isoliert sind.

7. Implantat (1) nach Anspruch 2, **dadurch gekennzeichnet, dass** die beiden Pole oder Elektroden (6) und (7) an sich gegenüberliegenden Außenseiten des Transponders (5) befestigt sind und mit diesem eine Einheit bilden.

8. Implantat (1) nach Anspruch 1, **dadurch gekennzeichnet, dass** die beiden Pole oder Elektroden (8) und (11) die in einer aus Silikon geformten Rille (9) eingebettet sind und durch diese Rille voneinander elektrisch getrennt und/oder zusätzlich gegeneinander isoliert sind.

9. Implantat (1) nach Anspruch 8, **dadurch gekennzeichnet, dass** die Rille 9 sich an der Innenseite (4b) der Schutzhülle (4) befindet.

10. Implantat (1) nach Anspruch 1, **dadurch gekennzeichnet, dass** die Hülle (2) und die Schutzhülle (4) aus Silikonmaterial besteht.

11. Implantat (1) nach Anspruch 1, **dadurch gekennzeichnet, dass** die elektrischen Pole oder die Elektroden (6), (7), (8) und (11) an der in Gebrauchsstellung rückwärtigen Wandung oder Rückseite (20) des Implantats (1) angeordnet oder befestigt ist.

12. Implantat (1) nach einem der Ansprüche 1 bis 11, **dadurch gekennzeichnet, dass** es an der die Außenelektrode (6) aufnehmenden Stelle oder Wandung eine Vertiefung für die Elektrode vorhanden ist und die Elektrode (6) in Gebrauchsstellung gegenüber der Oberfläche der Außenseite wenigstens teilweise oder derart eingesenkt ist, dass die Außenseite der Elektrode mit der Außenseite der ihn aufnehmenden Wandung bündig ist.

13. Implantat (1) nach einem der Ansprüche 1 bis 12, **dadurch gekennzeichnet, dass** an der Rückwand (20) des Implantats (1) eine Öffnung für das Einbringen der Implantats-Füllung und ein Verschluss dafür vorgesehen ist und dass der Transponder (5) mit den Elektroden (6) und (7) an oder in dem Verschluss angeordnet ist.
